# EUROPEAN PATENT SPECIFICATION

(11) **EP 3 701 049 B1**
(45) Date of publication and mention of the grant of the patent: **08.12.2021**
(21) Application number: 18788798.9
(22) Date of filing: 24.10.2018
(51) Int. Cl.: C12Q 1/6886

(54) **DIAGNOSIS AND/OR PROGNOSIS OF HER2-DEPENDENT CANCER USING MOESIN AS A BIOMARKER**
DIAGNOSE UND/ODER PROGNOSE VON HER2-ABHÄNGIGEM KREBS UNTER VERWENDUNG VON MOESIN ALS EINEN BIOMARKER
DIAGNOSTIC ET/OU PRONOSTIC DU CANCER DÉPENDANT AU HER2 À L'AIDE DE MOÉSINE EN TANT QUE BIOMARQUEUR

(30) Priority: 24.10.2017 EP 17306465
(43) Date of publication of application: 02.09.2020
(73) Proprietor: Université de Paris, 75006 Paris (FR); Institut National de la Santé et de la Recherche Médicale (INSERM), 75654 Paris Cedex 13 (FR); Centre National de la Recherche Scientifique (C.N.R.S.), 75794 Paris Cedex 16 (FR)
(72) Inventor: BOURDOULOUS, Sandrine, 93220 Gagny (FR); DOMINGOT, Anaïs, 75014 Paris (FR); FAURE, Camille, 91230 Montgeron (FR)
(74) Representative: Cabinet Beau de Loménie
(86) International application number: PCT/EP2018/079213
(87) International publication number: WO 2019/081608

(56) References cited:
- WO-A1-2015/161792
- FR-A1- 3 018 919
- US-A1- 2011 306 507
- EMMANUELLE CHARAFE-JAUFFRET ET AL: "Moesin expression is a marker of basal breast carcinomas", INTERNATIONAL JOURNAL OF CANCER, vol. 121, no. 8, 26 June 2007 (2007-06-26) , pages 1779-1785, XP055099832, US ISSN: 0020-7136, DOI: 10.1002/ijc.22923
- BARTOVA M ET AL: "Expression of ezrin and moesin in primary breast carcinoma and matched lymph node metastases", CLINICAL AND EXPERIMENTAL METASTASIS, SPRINGER NETHERLANDS, NL, vol. 34, no. 5, 17 June 2017 (2017-06-17), pages 333-344, XP036299974, ISSN: 0262-0898, DOI: 10.1007/S10585-017-9853-Y [retrieved on 2017-06-17]
- A PARADISO ET AL: "Nuclear NHERF1 expression as a prognostic marker in breast cancer", CELL DEATH & DISEASE, vol. 4, no. 11, 1 November 2013 (2013-11-01), pages e904-e904, XP055524052, DOI: 10.1038/cddis.2013.439

### SEQUENCE LISTING

<110> Université de Paris 5 René Descartes INSERM (Institut National de la Sante et de la Recherche Medicale) Centre National de la Recherche Scientifique (CNRS)
<120> Diagnosis and/or prognosis of HER2-dependent cancer using moesin as a biomarker
<130> 1H316870
<160> 7
<170> PatentIn version 3.5
<210> 1
   <211> 577
   <212> PRT
   <213> Homo sapiens
<400> 1 
Met Pro Lys Thr Ile Ser Val Arg Val Thr Thr Met Asp Ala Glu Leu
1               5                   10                  15

Glu Phe Ala Ile Gln Pro Asn Thr Thr Gly Lys Gln Leu Phe Asp Gln
            20                  25                  30

Val Val Lys Thr Ile Gly Leu Arg Glu Val Trp Phe Phe Gly Leu Gln
        35                  40                  45

Tyr Gln Asp Thr Lys Gly Phe Ser Thr Trp Leu Lys Leu Asn Lys Lys
    50                  55                  60

Val Thr Ala Gln Asp Val Arg Lys Glu Ser Pro Leu Leu Phe Lys Phe
65                  70                  75                  80

Arg Ala Lys Phe Tyr Pro Glu Asp Val Ser Glu Glu Leu Ile Gln Asp
                85                  90                  95

Ile Thr Gln Arg Leu Phe Phe Leu Gln Val Lys Glu Gly Ile Leu Asn
            100                 105                 110

Asp Asp Ile Tyr Cys Pro Pro Glu Thr Ala Val Leu Leu Ala Ser Tyr
        115                 120                 125

Ala Val Gln Ser Lys Tyr Gly Asp Phe Asn Lys Glu Val His Lys Ser
    130                 135                 140

Gly Tyr Leu Ala Gly Asp Lys Leu Leu Pro Gln Arg Val Leu Glu Gln
145                 150                 155                 160
 
His Lys Leu Asn Lys Asp Gln Trp Glu Glu Arg Ile Gln Val Trp His
                165                 170                 175

Glu Glu His Arg Gly Met Leu Arg Glu Asp Ala Val Leu Glu Tyr Leu
            180                 185                 190

Lys Ile Ala Gln Asp Leu Glu Met Tyr Gly Val Asn Tyr Phe Ser Ile
        195                 200                 205

Lys Asn Lys Lys Gly Ser Glu Leu Trp Leu Gly Val Asp Ala Leu Gly
    210                 215                 220

Leu Asn Ile Tyr Glu Gln Asn Asp Arg Leu Thr Pro Lys Ile Gly Phe
225                 230                 235                 240

Pro Trp Ser Glu Ile Arg Asn Ile Ser Phe Asn Asp Lys Lys Phe Val
                245                 250                 255

Ile Lys Pro Ile Asp Lys Lys Ala Pro Asp Phe Val Phe Tyr Ala Pro
            260                 265                 270

Arg Leu Arg Ile Asn Lys Arg Ile Leu Ala Leu Cys Met Gly Asn His
        275                 280                 285

Glu Leu Tyr Met Arg Arg Arg Lys Pro Asp Thr Ile Glu Val Gln Gln
    290                 295                 300

Met Lys Ala Gln Ala Arg Glu Glu Lys His Gln Lys Gln Met Glu Arg
305                 310                 315                 320

Ala Met Leu Glu Asn Glu Lys Lys Lys Arg Glu Met Ala Glu Lys Glu
                325                 330                 335

Lys Glu Lys Ile Glu Arg Glu Lys Glu Glu Leu Met Glu Arg Leu Lys
            340                 345                 350

Gln Ile Glu Glu Gln Thr Lys Lys Ala Gln Gln Glu Leu Glu Glu Gln
        355                 360                 365

Thr Arg Arg Ala Leu Glu Leu Glu Gln Glu Arg Lys Arg Ala Gln Ser
    370                 375                 380

Glu Ala Glu Lys Leu Ala Lys Glu Arg Gln Glu Ala Glu Glu Ala Lys
385                 390                 395                 400

Glu Ala Leu Leu Gln Ala Ser Arg Asp Gln Lys Lys Thr Gln Glu Gln
                405                 410                 415
 
Leu Ala Leu Glu Met Ala Glu Leu Thr Ala Arg Ile Ser Gln Leu Glu
            420                 425                 430

Met Ala Arg Gln Lys Lys Glu Ser Glu Ala Val Glu Trp Gln Gln Lys
        435                 440                 445

Ala Gln Met Val Gln Glu Asp Leu Glu Lys Thr Arg Ala Glu Leu Lys
    450                 455                 460

Thr Ala Met Ser Thr Pro His Val Ala Glu Pro Ala Glu Asn Glu Gln
465                 470                 475                 480

Asp Glu Gln Asp Glu Asn Gly Ala Glu Ala Ser Ala Asp Leu Arg Ala
                485                 490                 495

Asp Ala Met Ala Lys Asp Arg Ser Glu Glu Glu Arg Thr Thr Glu Ala
            500                 505                 510

Glu Lys Asn Glu Arg Val Gln Lys His Leu Lys Ala Leu Thr Ser Glu
        515                 520                 525

Leu Ala Asn Ala Arg Asp Glu Ser Lys Lys Thr Ala Asn Asp Met Ile
    530                 535                 540

His Ala Glu Asn Met Arg Leu Gly Arg Asp Lys Tyr Lys Thr Leu Arg
545                 550                 555                 560

Gln Ile Arg Gln Gly Asn Thr Lys Gln Arg Ile Asp Glu Phe Glu Ser
                565                 570                 575
 Met
<210> 2
   <211> 17
   <212> DNA
   <213> Artificial Sequence
<220>
   <223> PCR Primer (Forward HER2)
<400> 2
   gccgcagtga gcaccat 17
<210> 3
   <211> 19
   <212> DNA
   <213> Artificial Sequence
<220>
   <223> PCR Primer (Reverse HER2)
<400> 3
   cgcagcttca tgtctgtgc 19
<210> 4
   <211> 27
   <212> DNA
   <213> Artificial Sequence
<220>
   <223> PCR Primer (Forward MSN)
<400> 4
   ggaatgagac aggacctagg atatctt 27
<210> 5
   <211> 27
   <212> DNA
   <213> Artificial Sequence
<220>
   <223> PCR Primer (Reverse MSN)
<400> 5
   ggaatgagac aggacctagg atatctt 27
<210> 6
   <211> 21
   <212> DNA
   <213> Artificial Sequence
<220>
   <223> PCR Primer (Forward GAPDH)
<400> 6
   gatccctcca aaatcaagtg g 21
<210> 7
   <211> 22
   <212> DNA
   <213> Artificial Sequence
<220>
   <223> PCR Primer (Reverse GAPDH)
<400> 7
   gcaaatgagc cccagccttc tc 22

## Description

### BACKGROUND OF THE INVENTION

Breast cancer is the most common cancer in women worldwide, with nearly 1.7 million new cases diagnosed In 2012 (second most common cancer overall). This represents about 12% of all new cancer cases and 25% of all cancers in women. About half the breast cancer cases and 60% of the deaths are estimated to occur in economically developing countries. The rate of incidence observed in France is among the strongest in Europe and is in constant increase.

About 20 to 30% of primary human breast cancers are due to the deregulated expression of HER2: it represents approximately 8,000 patients a year in France and 450,000 patients a year worldwide. HER2 is a well-known member of the human epidermal growth factor receptor (HER/EGFR/ERBB) family. ErbB2 overexpression is associated with a poor diagnosis: tumors with deregulated HER2 have been shown to grow faster to be more aggressive and to be less sensitive to chemotherapy or to hormonotherapy. HER2 deregulation is also associated with disease recurrence. Then, so called HER2-dependent cancer constitute a very specific group of cancer of an utmost interest in public health. Indeed, only 25% of the treated patients respond to the actual therapies. The actual strategies aiming at targeting the extracellular domain of HER2 (anti-HER2 antibody therapies such as Herceptin/Trastuzumab, Pertuzumab and the recently developed Trastuzumab emtansine from Genentech, USA) or the kinase activity of the receptor (small molecule tyrosine kinase inhibitors, such as Lapatinib/Tykerb, GSK, USA) have proven to exhibit limited actions.

In particular, these molecules have no potent action on the mutated and truncated forms of HER2. Concerning Trastuzumab, 66% to 88 % of treated patients never respond to treatment (i.e. present a "primary resistance") and among the one-third of the treated patients that respond to this agent, a disease progression on average in less than one year (i.e. develop an "acquired resistance") is generally observed. The efficacy is limited by the development of therapeutic resistance mainly attributed to the expression of p95HER-2, as this highly active truncated form of HER2 lacks the recognition site for Trastuzumab.

Generally, HER2 (over)expression is established as a marker of poor prognosis in breast cancer. Different techniques have been developed to evaluate if a cancer/patient is "positive" for HER2 (i.e. HER2+) or "negative" for HER2 (i.e. HER2-), such as fluorescence *in situ* hybridization (FISH) test or immunohistochemistry (IHC test). The publication of Charafe-Jauffret et al. (INTERNATIONAL JOURNAL OF CANCER, vol. 121, no. 8, 26 June 2007 (2007-06-26), pages 1779-1785 2007) discloses that Moesin expression is correlated with a basal phenotype both in sporadic breast cancers and in other specific types of basal cancers.

The inventors have identified a strong functional link between moesin and HER2 genes and found that that diagnosis of HER2-dependent cancer or a poor prognosis of HER2-dependent cancer is specifically associated with a low level of moesin in HER2+ subjects. This finding makes it possible to develop a new method for diagnosing and/or prognosticating HER2-dependent cancer in a subject.

### SUMMARY OF THE INVENTION

The inventors therefore propose here a method for the diagnostic and/or prognosis wherein the amount of moesin in the sample from a subject compared to a reference value represents the prediction of HER2-dependent cancer or represents a prognosis for HER2-dependent cancer, indicating that the subject has a risk or no of having and/or developing HER2-dependent cancer.

The invention relates to a method for diagnosing and/or prognosticating HER2-dependent cancer in a subject, comprising:
a) measuring the amount of moesin in a sample from the subject;
b) comparing the amount of moesin measured in step a) to a reference value;
c) finding a deviation or no deviation of the amount of moesin measured in step a) from the reference value; and
d) attributing said finding of deviation or no deviation to a particular diagnosis and/or prognosis of HER2-dependent cancer in the subject.

The invention also relates to a method for monitoring a change in the diagnosis and/or prognosis of HER2-dependent cancer in a subject, comprising:
a) applying the method of the invention to the subject at one or more successive time points, whereby the diagnosis and/or prognosis of HER2-dependent cancer in the subject is determined at said successive time points;
b) comparing the diagnosis and/or prognosis of HER2-dependent cancer in the subject at said successive time points as determined in step a); and
c) finding the presence or absence of a change between the diagnosis of HER2-dependent cancer in the subject at said successive time points as determined in step a).

The invention also relates to a kit for determining the diagnostic and/or the prognosis of HER2-dependent cancer in a subject suffering thereof, comprising:
- means for measuring the amount of moesin in the sample from the subject; and
- a reference value of the amount of moesin or means for establishing said reference value, said reference value representing a known diagnosis and/or prognosis of HER2-dependent cancer.

The invention also relates to the use of a kit comprising:
- means for measuring the amount of moesin in the sample from the subject; and
- a reference value of the amount of moesin or means for establishing said reference value, said reference value representing a known diagnosis and/or prognosis of HER2-dependent cancer
for determining the diagnostic and/or the prognosis of HER2-dependent cancer in a subject suffering thereof.

The invention also relates to the use of the two probes SEQ ID NO: 4 and SEQ ID NO: 5 for measuring the mRNA level of moesin by qRT-PCR for determining the diagnostic and/or the prognosis of HER2-dependent cancer in a human subject suffering thereof.

### DETAILED DESCRIPTION OF THE INVENTION

### Definitions

The terms "diagnosing" or "diagnosis" and "prognosticating" or "prognosis", as used herein, are used in the broadest sense, and are commonly used and are well-understood in medical and clinical practice. By means of further explanation and without limitation, "prognosticating" or "prognosis" refers to the determination of probability, risk or possibility of developing HER2-dependent cancer in a subject. By means of further explanation and without limitation, "diagnosing" or "diagnosis" refers to the determination of a probability or a possibility of having HER2-dependent cancer in a subject.

The term "HER2", as used herein, refers to "Human Epidermal growth factor Receptor 2" which is a protein member of the human epidermal growth factor receptor family. "HER2" is also frequently called "ErbB2". "ErbB2" and "HER2" are used interchangeably in the present invention.

The term "HER2-dependent cancer" or "HER2 positive cancer" or "HER2+ cancer", as used herein, refers to cancer involving exacerbated HER2 activation. In particular, the term "HER2-dependant cancer" refers to any cancer case for which cancer cells exhibiting a deregulation of HER2 gene have been identified, in opposition to "HER2-independent cancer" or "HER2 negative cancer" or "HER2- cancer". More particularly said deregulation can correspond to an amplification of HER2 gene. This amplification can be detected at the genetic level, or at the protein level. For example, guidelines emitted by the American society of Clinical Oncology/College of American Pathologists (ASCO/CAP) for breast cancer set several cut-offs for determining the ErbB2 status of breast cancer. These guidelines prescribe that a cancer should be considered as "HER2-dependent" if, for the primary site and if possible for the metastatic site:
- a uniform and intense membrane staining of more than 30 % of invasive tumor cells is observed in immunochemistry (IHC), or
- i) a FISH amplified ratio of HER2 to CEP17 (chromosome 17 centromere) superior or equal to 2 (dual probe testing) or ii) a FISH amplified ratio of HER2 to CEP17 (chromosome 17 centromere) inferior to 2 (dual probe testing) with an average HER2 copy number of at least 6 copies per nucleus (single probe testing) is determined, or iii) a single probe average of at least 6 signals for the HER2 copy number per cells.

Besides, a cancer is considered as "HER2-independent" when, for the primary site and if possible for the metastatic site:
- in IHC, no staining or a weak incomplete membrane staining, or a weak but complete membrane staining is observed in less than 10% of cells, or
- the FISH HER2/CEP17 ratio inferior to 2 with an average copy number of HER2 inferior to 4 signals per cells is noticed (dual probe testing), or an average copy number of HER2 inferior to 4 signals per cell is noticed (in cases where a single probe is used).

The HER2 status will be considered as equivocal (then a new test should be performed) when, for the primary site and, if possible, for the metastatic site:
- in IHC, i) an incomplete labelling of circumferential membrane and/or weak/moderate labelling is noticed but within superior to 10% of the invasive tumor cells or ii) a complete and intense labelling of circumferential membrane is noticed but for 10% or less of the invasive tumor cells, or
- the FISH HER2/CEP17 ratio inferior to 2 with an average copy number of HER2 of at least 4 signals but less than 6 signals per cell is noticed (dual probe testing), or an average copy number of HER2 of at least 4 signals but less than 6 signals per cell is noticed (in cases where a single probe is used).

Deregulation of HER2 gene can also correspond to activating mutations in HER2 gene disregarding its copy number, leading to an increase of the tyrosine kinase activity of HER2. For example, said activating mutations can be V659E, G309A, D769H, D769Y, V777L, P780ins, V842I,R896C, K753E or L755S and can be detected by Polymerase Chain Reaction or any sequencing technique [Bose et al. Cancer Discov. 2013, 3(2), 224-237; Zuo et al. Clin Cancer Res 2016, 22(19), 4859-4869]. Also, both an amplification of HER2 gene and a somatic activating mutation can be detected in the same case of cancer.

Well known molecular biology tests other than Fish or IHC, using negative and positive control cells with an established HER2 status, can be used for determining the HER2 status of a cancer by way of comparison, as for example Enzyme-Linked Immunosorbent Assays, Western blotting assays, Polymerase Chain Reaction, etc.

Preferably, the HER2-dependent cancer according to the invention is selected from the group consisting of breast cancer, female genital tract cancer, such as endometrial cancer, uterine cancer or ovarian cancer, bladder cancer, anal cancer, colorectal cancer, in particular uterine serous cancer, such as uterine papillary serous carcinoma, lung cancer, in particular non-small-cell lung cancer, liver cancer, kidney cancer, gastroesophageal cancer, stomach cancer, pancreas cancer and gastric cancer. In a preferred embodiment, the HER2-dependent cancer may be HER2+ breast cancer, HER2+ ovarian cancer, HER2+ bladder cancer, HER2+ colorectal cancer, HER2+ uterine papillary serous carcinoma and HER2+ gastric cancer, preferably HER2+ breast cancer.

For the purposes of the invention, the term "marker" (or "biomarker") is an entity, such as a protein or a ribonucleic acid (RNA), which can be used for the diagnosis and/or prognosis of HER2-dependent cancer. According to the invention, the biomarker is moesin, i.e. the protein itself or a RNA coding said moesin, such as a mRNA. For the purposes of the invention, the biomarker is preferably a protein.

Moesin (for membrane-organizing extension spike protein) is a protein that in humans is encoded by the MSN gene. Moesin is a member of the ERM protein family which consists of three closely related proteins, Ezrin, Radixin and Moesin, that play a key role in a large number of important physiological processes such as cell morphogenesis, adhesion or migration, as they provide a regulated linkage between membrane proteins and the cortical cytoskeleton and trigger signal-transduction pathways. Exemplary moesin includes, without limitation, human moesin having the amino acid sequence SEQ ID NO: 1 (Uniprot accession number: P26038).

The term "subject" or "patient", as used herein, refers to a human or non-human mammal (such as a rodent (mouse, rat), a feline, a canine, or a primate) affected or likely to be affected with HER2-dependent cancer. Preferably, the subject is a human, man or woman. Preferably, the subject is HER2+.

The term "HER2+ subject", as used herein, refers to a subject having a cancer being positive for the expression and/or activation of HER2. Different techniques may be used for assaying HER2 expression, for example fluorescence *in situ* hybridization (FISH) test, chromogenic *in situ* hybridization, real-time quantitative polymerase-chain-reaction (qRT-PCR) or immunohistochemistry (IHC) test.

The term "sample" or "biological sample", as used herein, includes any biological specimen obtained from a subject, such as a tumor sample or a body fluid sample. A body fluid sample may be selected from the group consisting of blood, serum plasma, saliva, urine, amniotic fluid, breast milk, bronchial lavage, cerebrospinal fluid, peritoneal fluid, seminal fluid, tears and pleural fluid, preferably blood. A tumor sample may include biopsies, such as tumor biopsies.

The term "reference value" or "reference profile" means a value or profile representing (i) a diagnosis of no HER2-dependent cancer, (ii) a diagnosis of HER2-dependent cancer, (iii) a good prognosis of HER2-dependent cancer or (iv) a bad prognosis of HER2-dependent cancer. For example, a reference value for the amount of moesin may be obtained as follows:
a) measuring the amount of moesin in:
   a1) one or more samples from one or more subjects not having HER2-dependent cancer, or
   a2) one or more samples from one or more subjects having HER2-dependent cancer, and
b) storing the quantity of moesin
   b1) as measured in (a1) as the reference value representing the diagnosis and/or prognosis of no HER2-dependent cancer, or
   b2) as measured in (a2) as the reference value representing the diagnosis and/or prognosis of HER2-dependent cancer.

A reference value also encompasses a reference range.

The terms "amount", "quantity", and "level" are synonymous and generally well-understood in the art. The terms as used herein may particularly refer to an absolute quantification of a biomarker in a sample, or to a relative quantification of a biomarker in a sample (i.e. relative to another value such as relative to a reference value as taught herein), or to a range of values indicating a base-line expression of the biomarker. These values or ranges can be obtained from a single patient or from a group of patients. An absolute quantity of a biomarker in a sample may be advantageously expressed as weight or as molar amount, or more commonly as a concentration. A relative quantity of a biomarker in a sample may be advantageously expressed as an increase or a decrease relative to (i.e. compared to) another value, such as relative to (i.e. compared to) a reference value.

The term "deviation" of a first value from a second value may generally encompass any direction, such as increase or decrease of a first value compared to a second value.

The term "treating" or "treatment" means reversing, alleviating, inhibiting the progress of, or preventing the disorder or condition to which such term applies, or one or more symptoms of such disorder or condition. In particular, the treatment of the disorder may consist in destroying, depleting or inhibiting the proliferation of cancer cells. Most preferably, such treatment leads to the complete depletion of cancer cells.

### Diagnosis and/or prognosis

The present invention results from the advantages highlighted by the inventors that the increase or decrease of the amount of moesin makes it possible to diagnose and/or prognose HER2-dependent cancer. The resulting applications are described below.

The invention relates to a method for diagnosing and/or prognosticating HER2-dependent cancer in a subject, comprising:
a) measuring the amount of moesin in a sample from the subject;
b) comparing the amount of moesin measured in step a) to a reference value;
c) finding a deviation or no deviation of the amount of moesin measured in step a) from the reference value; and
d) attributing said finding of deviation or no deviation to a particular diagnosis and/or prognosis of HER2-dependent cancer in the subject.

The methods of the invention may be used in individuals who have not yet been diagnosed as having HER2-dependent cancer (for example, preventative screening), or who have been diagnosed as having HER2-dependent cancer, or who are suspected of having HER2-dependent cancer (for example, display one or more symptoms characteristic of HER2-dependent cancer), or who are at risk of developing HER2-dependent cancer. The methods may also be used to detect various stages of progression or severity of HER2-dependent cancer.

### Step a)

Step a) is the measure of the amount of moesin in a sample from the subject.

The amount of moesin may be measured by any suitable technique known in the prior art. In some embodiments, the amount of moesin a may be measured using a binding agent capable of specifically binding to moesin and/or to fragments thereof. The binding agent may be an antibody, an aptamer, a protein, a peptide or a small molecule. For example, the amount of moesin is measured using an immunoassay technology, such as enzyme-linked immunosorbent assay (ELISA), Immunoradiometric Assay (IRMA) or radioimmunoassay (RIA), or using a mass spectrometry analysis method or using a chromatography method, or using a combination of said methods. In some other embodiments, the amount of moesin may be determined by the measure of the moesin mRNA using any suitable technique, such as qRT-PCR or digital molecular barcoding technology for RNA or DNA microarrays.

### Step b) & c)

Step b) and c) is the comparison of the amount of moesin measured in step a) to a reference value and the finding of a deviation or no deviation of the amount of moesin measured in step a) from the reference value.

A deviation of the amount of moesin may encompass a decrease of the amount of moesin compared to the reference value or an increase of the amount of moesin compared to the reference value.

For example, a deviation of the amount of moesin compared to the reference value may encompass a decrease of at least about 10% (about 0.9-fold or less), or by at least about 20% (about 0.8-fold or less), or by at least about 30% (about 0.7-fold or less), or by at least about 40% (about 0.6-fold or less), or by at least about 50% (about 0.5-fold or less), or by at least about 60% (about 0.4-fold or less), or by at least about 70% (about 0.3-fold or less), or by at least about 80% (about 0.2-fold or less), or by at least about 90% (about 0.1-fold or less), or more compared to the reference value.

For example, a deviation of the amount of moesin compared to the reference value may encompass an increase of at least about 10% (about 1.1-fold or more), or by at least about 20% (about 1.2-fold or more), or by at least about 30% (about 1.3-fold or more), or by at least about 40% (about 1.4-fold or more), or by at least about 50% (about 1.5-fold or more), or by at least about 60% (about 1.6-fold or more), or by at least about 70% (about 1.7-fold or more), or by at least about 80% (about 1.8-fold or more), or by at least about 90% (about 1.9-fold or more), or by at least about 100% (about 2-fold or more), or by at least about 150% (about 2.5-fold or more), or by at least about 200% (about 3-fold or more), or by at least about 500% (about 6-fold or more), or by at least about 900% (about 10-fold or more), or more, compared to a reference value.

In a preferred embodiment, the amount of the moesin in the sample from the subject is at least 0.5-fold decreased compared to the reference value, more preferably at least 0.7-fold decreased.

In another preferred embodiment, the amount of moesin in the sample from the subject is at least 1.5-fold increased compared to the reference value, more preferably at least 2-fold increased.

In another preferred embodiment, the amount of moesin in the sample from the subject is equal to the reference value.

### Step d)

Step d) is the attribution of the finding of deviation or no deviation in step c) to a particular diagnosis and/or prognosis of HER2-dependent cancer in the subject.

A deviation which is an increase of the amount of moesin compared to the reference value represents the diagnosis of no HER2-dependent cancer or represents a good prognosis for HER2-dependent cancer. Accordingly, a higher amount of moesin in the sample from the subject compared to the reference value indicates that the subject has no HER2-dependent cancer or indicates a long-term survival of said subject.

A deviation which is a decrease of the amount of moesin compared to the reference value represents the diagnosis of HER2-dependent cancer or represents a bad prognosis for HER2-dependent cancer. Accordingly, a lower amount of moesin in the sample from the subject compared to the reference value indicates that the subject has HER2-dependent cancer and/or indicates a short-term survival of said subject.

When no deviation is found between the amount of moesin and a reference value, the absence of such deviation is indicative of or may be attributed to the conclusion that the diagnosis and/or prognosis of HER2-dependent cancer is substantially the same as that represented by the reference value.

The methods of the invention may also be combined with the assessment of one or more further biomarkers relevant for HER2-dependent cancer.

The one or more biomarkers may be chosen from the group consisting of ER/PR (Estrogen Receptor/Progesterone Receptor), HER2, p95HER2, KI67, TOP2A (Topoisomerase 2-alpha), BCL2 (B-cell lymphoma 2), GSTM1 (Glutathione S-Transferase Mu 1), BAG1, Cyclin B1, cathepsin L2, CD68 and TP53.

### A method for monitoring a change in the diagnosis and/or prognosis

The method for diagnosing and/or prognosticating HER2-dependent cancer in a subject may also be used to detect response of HER2-dependent cancer to prophylactic or therapeutic treatments or other interventions, e.g. by performing the methods at different time points during said prophylactic or therapeutic treatment or other intervention.

The invention therefore relates to a method for monitoring a change in the diagnosis and/or prognosis of HER2-dependent cancer in a subject, comprising:
a) applying the method of any of claims 1 to 10 to the subject at one or more successive time points, whereby the diagnosis and/or prognosis of HER2-dependent cancer in the subject is determined at said successive time points;
b) comparing the diagnosis and/or prognosis of HER2-dependent cancer in the subject at said successive time points as determined in step a); and
c) finding the presence or absence of a change between the diagnosis of HER2-dependent cancer in the subject at said successive time points as determined in step a).

In some embodiments, said change in the diagnosis and/or prognosis of HER2-dependent cancer in the subject is monitored in the course of a medical treatment of said subject.

The medical treatment may be a prophylactic treatment or a therapeutic treatment, preferably an antitumoral treatment. The antitumoral treatment may be any treatment known for the treatment of said HER2-dependent cancer. For example, the antitumoral treatment may aim at targeting the extracellular domain of HER2, such as anti-HER2 antibody therapies, for example Herceptin/Trastuzumab, Pertuzumab and Trastuzumab emtansine (Genentech, USA); or the antitumoral treatment may aim at targeting the kinase activity of HER2, such as small molecule tyrosine kinase inhibitors, for example, Neratinib, Tucatinib or, Lapatinib/Tykerb (GSK, USA). The antitumoral treatment may also be a taxane-based chemotherapy, such as paclitaxel.

### Kit

The invention also relates to a kit for determining the diagnostic and/or the prognosis of HER2-dependent cancer in a subject suffering thereof, comprising:
- means for measuring the amount of moesin in the sample from the subject; and
- a reference value of the amount of moesin or means for establishing said reference value, said reference value representing a known diagnosis and/or prognosis of HER2-dependent cancer.

The invention also relates to the use of a kit comprising:
- means for measuring the amount of moesin in the sample from the subject; and
- a reference value of the amount of moesin or means for establishing said reference value, said reference value representing a known diagnosis and/or prognosis of HER2-dependent cancer
for determining the diagnostic and/or the prognosis of HER2-dependent cancer in a subject suffering thereof.

In some embodiments, means may comprise a binding agent capable of specifically binding to moesin and/or to fragments thereof. The binding agent may be an antibody, an aptamer, a protein, a peptide or a small molecule.

In some other embodiments, means may comprise specific amplification primers and/or probes for the specific quantitative amplification of moesin mRNA, for example the primers and/or probes are SEQ ID NO: 4 and SEQ ID NO: 5.

The invention also relates to the use of primers and/or probes SEQ ID NO: 4 and SEQ ID NO: 5 for the specific quantitative amplification of moesin mRNA for determining the diagnostic and/or the prognosis of HER2-dependent cancer in a human subject suffering thereof.

### Method of treatment

As described above, the present invention is useful to detect response of HER2-dependent cancer to prophylactic or therapeutic treatments or other interventions, e.g. by performing the methods at different time points during said prophylactic or therapeutic treatment or other intervention.

Similarly, the present invention also relates to a method for treating HER2-dependent cancer in a subject, comprising:
(i) the implementation of a diagnostic or prognostic method according to the invention, and
(ii) when the method indicates that the subject has HER2-dependent cancer and/or indicates a short-term survival of said subject, institute appropriate treatment in said subject.

In one particular embodiment, said appropriate treatment is one or more medicines selected from a prophylactic treatment or a therapeutic treatment, preferably an antitumoral treatment aiming at targeting the extracellular domain of HER2, such as anti-HER2 antibody therapies, for example Herceptin/Trastuzumab, Pertuzumab and Trastuzumab emtansine (Genentech, USA) or aiming at targeting the kinase activity of HER2, such as small molecule tyrosine kinase inhibitors, for example Neratinib, Tucatinib or Lapatinib/Tykerb (GSK, USA).

### FIGURES

### Brief summary of the figures:

**Figure 1** represents the quantification of western blot analyses of the level of expression of Ezrin and Radixin (Figure 1A, left panel) and HER2 and moesin (Figure 1A, right panel) in some breast cancer cell lines with different HER2 status. Figure 1a shows a strong inverse correlation between expression/activation levels of HER2 and expression levels of Moesin. This result was further confirmed at the mRNA level by qRT-PCR analysis performed on 25 breast cancer cells lines with various HER2 status (Figure 1B).
   MSN^{hi} means high expression of MSN.
   MSN^{lo} means low expression of MSN.
**Figure 2** represents Kaplan-Meier overall survival analyses of 30 patients with HER2+ breast cancer (HER2+ BC) or 130 patients with HER2- breast cancer (HER2- BC) depending on moesin level of expression. BC means "Breast Cancer".
**Figure 3** represents the inverse correlation between the expression of HER2 and MSN mRNA data (Agilent microarray) from 529 tumor samples from patients with breast invasive carcinoma of TCGA cohort.
**Figure 4** represents Kaplan-Meier overall survival analyses of patients from METABRIC cohort with HER2+ breast cancer (n=236, HER2+ BC) or HER2- breast cancer (n=1668, HER2- BC) depending on moesin level of expression.

### EXAMPLES

### Example 1: Methods

### METHODS:

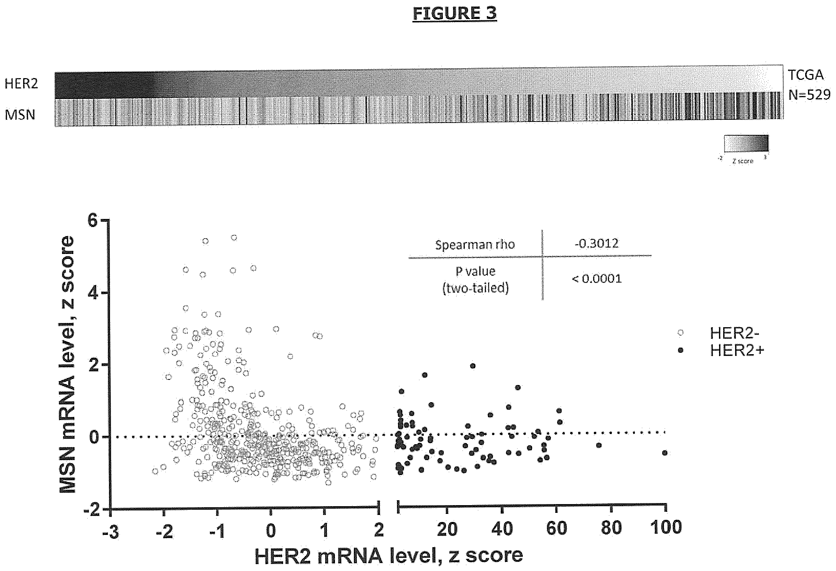

*Datasets and mRNA expression analysis* MSN and HER2 mRNA expression profiles in 529 samples from patients with breast invasive carcinoma of TCGA cohort were collected from cbioportal database (http://www.cbioportal.org). A color-coded representation of their expression is shown in the top panel whereas a scatter plot is shown in the bottom panel. Correlation analysis was then done by Spearman rho coefficient calculation using GraphPad prism software (figure 3).

### Datasets and mRNA expression analyses

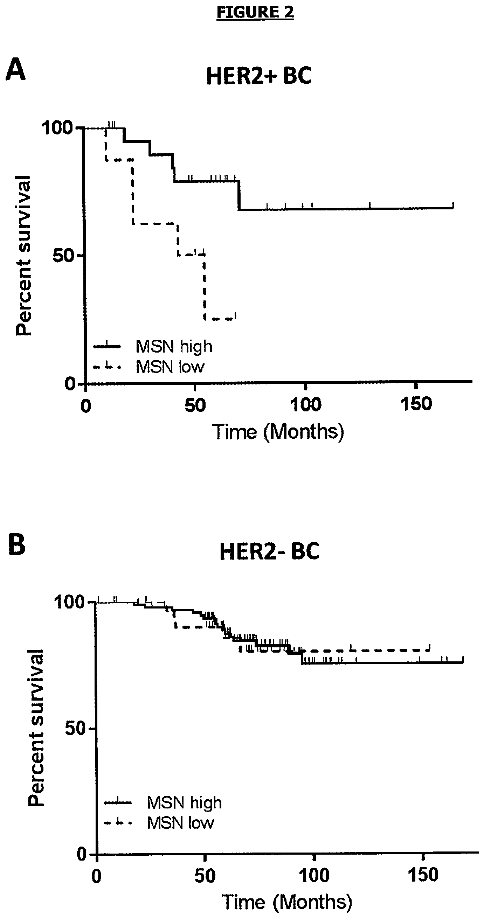

Overall survival of breast cancer patients was assessed with the online Kaplan-Meier plotter (http://kmplot.com/analysis/). Patients (Gene expression data and survival information originated from 1,809 patients from GEO (Affymetrix HGU133A and HGU133+2 microarrays) were divided into two groups according to lower quartile of moesin expression levels. Analyses were either restricted to HER2 positive status ((ICH) only and molecular subtype HER2+ER, n=30) or to HER2- (ER+/-, n=130) breast cancer patients. The two patient cohorts are compared by a Kaplan-Meier survival plot, and the hazard ratio with 95% confidence intervals and logrank P value are calculated. Each database is updated biannually (Figure 2).

Overall survival of breast cancer patients from METABRIC cohort was also analyzed based on their clinical and transcriptomic profiles obtained in the cbioportal database (http://www.cbioportal.org/). 236 HER2+ breast cancer patients or 1668 HER2- breast cancer patients were divided into two groups according to median moesin expression levels. The two patient cohorts are compared by a Kaplan-Meier survival plot, and the hazard ratio with 95% confidence intervals and Log-rank (Mantel-Cox) test value were calculated.

### Cell lines

Lysates from breast cancer cell line with different HER2 status were analyzed UACC-812 (ATCC^{®} CRL-1897^{™}), MDA-MB-361 (ATCC^{®} HTB-27^{™}), ZR-75-30 (ATCC^{®} CRL-1504'"), HCC202 (ATCC^{®} CRL-2316^{™}) and SK-BR-3 [SKBR3] (ATCC^{®} HTB-30^{™}) and were obtained from Dr. Emmanuelle Charafe-Jauffret (Institut Paoli-Calmettes, Aix Marseille University).

### Reagents

Primary antibodies used were anti-HER2 (ab2428, Abcam; 1:1000), anti-clathrin (BD Biosciences #610500; 1:1000). Secondary antibodies were anti-rabbit (#NA9340-1ML GE Healthcare) or mouse IgG (#NA9310-1ML GE Healthcare) coupled with HRP (GE Healthcare; 1:5000). The anti-MSN was a gift from Dr. Paul Mangeat (Université de Montpellier) and was used at a 1:1000 dilution.

### Western Blot

Lysates were denaturated with Laemmli buffer 10 min at 95°C and proteins were separated on 10% SDS/polyacrylamide gels (ProSieve, Lonza) in gradient buffer (100 mM Tris, 100 mM Tricine, 0.1% SDS), and transferred onto nitrocellulose membranes (Whatman^{®}) using transfert buffer (20% EtOH, 25 mM Tris, 192 mM Glycine pH 8,5) according to standard protocols. Membranes were blocked using 3% BSA in TBST buffer (140 mM NaCl, 3 mM KCI, 25 mM Tris, 0.1% Tween) 30 min at room temperature, and incubated over night at 4°C with primary antibodies. Horseradish peroxidase-conjugated anti-rabbit or anti-mouse antibody was used as a secondary antibody. Detection was performed using the SuperSignal West Pico Chemiluminescent Substrate (Pierce) on the FUSION-FX7- SPECTRA acquisition system (Vilber Lourmat).

### RNA extraction, cDNA preparation and quantitative real time RT-PCR analysis

Total RNA was extracted from cells using Tri Reagent^{®} (Sigma T9424). The concentration and integrity of total RNA were measured using a NanoDrop 2000 spectrophotometer (Thermo Scientific, Wilmington, DE). Reverse transcription reactions were performed with 200 ng total RNA using SuperScript^{™} II RT after RNaseOUT^{™} treatment (Thermo Scientific, Wilmington, DE). Real time quantitative PCR (qRT-PCR) was performed in 10 µl using SYBR Green I Master kit (Roche Life Science) according to the manufacturer's instructions on a LightCycler 480 Instrument II.

The PCR primers were:

**HER2**

| | |
|---|---|
| Forward HER2 | 5'-GCCGCAGTGAGCACCAT-3 (SEQ ID NO: 2) |
| Reverse HER2 | 5'-CGCAGCTTCATGTCTGTGC-3' (SEQ ID NO: 3) |

**MSN**

| | |
|---|---|
| Forward MSN | 5'- GGAATGAGACAGGACCTAGGATATCTT-3' (SEQ ID NO: 4) |
| Reverse MSN | 5'- GGAATGAGACAGGACCTAGGATATCTT-3' (SEQ ID NO: 5) |

**GAPDH**

| | |
|---|---|
| Forward GAPDH | 5'-GATCCCTCCAAAATCAAGTGG-3' (SEQ ID NO: 6) |
| Reverse GAPDH | 5'-GCAAATGAGCCCCAGCCTTCTC-3' (SEQ ID NO: 7) |

The PCR conditions were: 95°C, 10 s; 60°C, 10 s; 72°C, 15 s. At the conclusion of each run, a melt curve analysis was performed to ensure that a single product had been synthesized. The relative expression of HER2, normalized to GAPDH, was calculated using the 2-ΔΔCt method. The HER2 and MSN mRNA level analysis performed on 25 breast cancer cell lines with various HER2 status.

### Example 2 : influence of Moesin expression on HER2 expression

### Materials and Methods

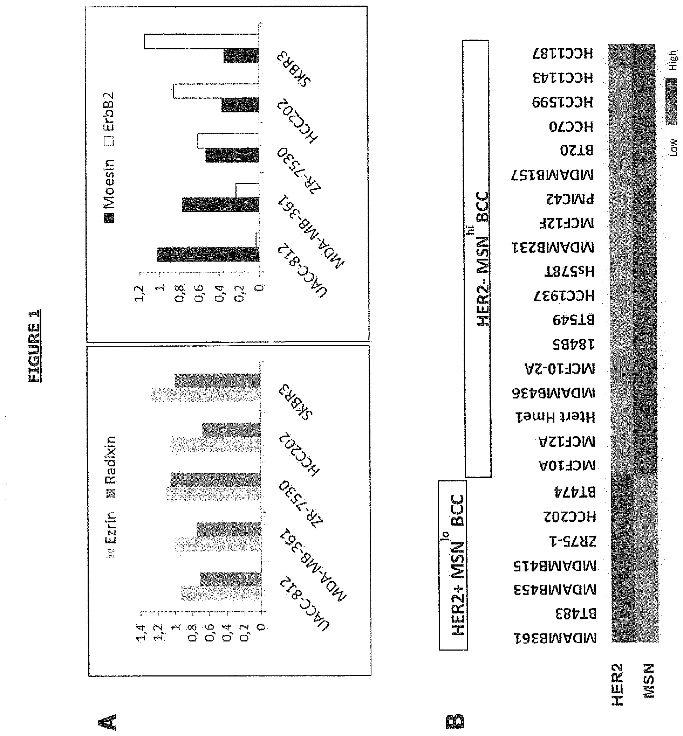

HER2 and moesin expression levels were analyzed by western blot and quantified in some breast cancer cell lines with different HER2 status (UACC812, MDAMB361, ZR-7530, HCC202 and SKBR3) (Figure 1A).

HER2 and moesin mRNA expression levels were obtained from agilent microarray data and collected from 529 patients with breast invasive carcinoma of TCGA cohort (http://www.cbioportal.org). A color-coded representation of their expression is shown in the top panel whereas a scatter plot is shown in the bottom panel. Patients with low level of HER2 expression (zscore<2) are shown in light grey whereas patients exhibiting high level of HER2 expression (zscore≥2) are shown in black. Correlation between HER2 and MSN mRNA expression was then assessed by Spearman rho coefficient calculation (r=-0.3012, P<0.0001) (Figure 3).

The impact of moesin level of expression on overall survival of patients with breast cancers was analyzed using the online Kaplan-Meier plotter (http://kmplot.com/analysis /) for breast cancers. Patients were divided into two groups according to lower quartile of moesin expression levels. Analyses were either restricted to HER2 positive status (HER2+ by Immuno-histo chemistry (IHC) on molecular subtype HER2+ER), Figure 2A) or restricted to HER2- breast cancer patients (Figure 2B).

### Results

Analysis conducted *in vitro* on different breast cancer cell lines indicated a strong inverse correlation between expression/activation levels of HER2 and expression levels of moesin (Figure 1).

This is also found *in vivo,* while analyzing tumor samples from 529 patients with breast invasive carcinoma it is observed clearly that Moesin expression is low in patients with high HER2 expression (Figure 3). Spearman correlation test indeed revealed a strong and highly significant inverse correlation between mRNA expression levels of HER2 and Moesin (r=-0.3012, P<0.0001) (Figure 3).

Kaplan-Meier survival analysis on breast cancer data revealed that low moesin expression levels among 30 patients diagnosed with a HER2+ breast cancer were associated with a significantly shorter overall survival and a bad prognosis compared to patients with high Moesin expression (42 vs. >150 months, P=0,0239; 75% death compared to <30%) (Figure 2A). However, this was specific of HER2+ breast cancers since level of Moesin expression did not alter survival profile of HER2- breast cancer patients (Figure 2B).

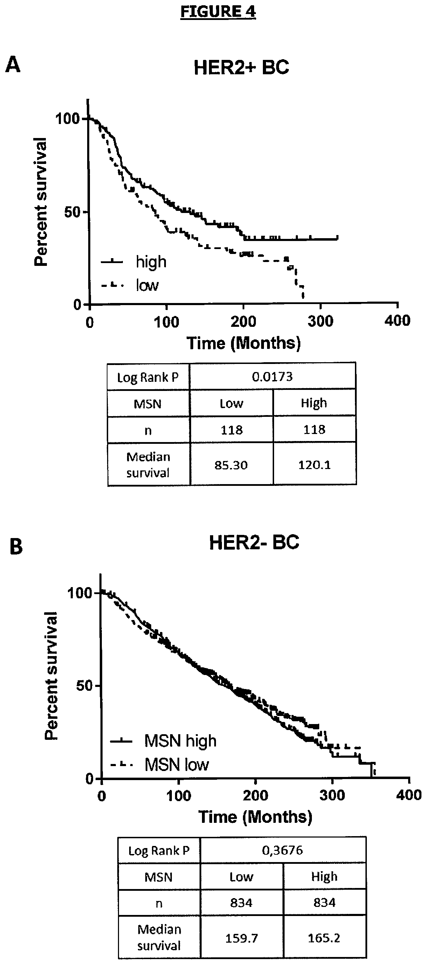

Analyzing data from METABRIC cohort supports these findings. Low moesin expression levels among 236 patients diagnosed with a HER2+ breast cancer are found associated with a significantly shorter overall survival and a bad prognosis compared to patients with high moesin expression (85.3 vs. 120.1 months, P=0.0173; 68% death compared to 55%) (Figure 4A). Importantly, this is specific of HER2+ breast cancers since level of moesin expression does not allow to segregate HER2- breast cancer patients according their overall survival expectancy (1168 Her2- patients P=0.3676, Figure 4B).

### Conclusions

These results demonstrate an inverse correlation between HER2 and MSN repression *in vitro* (in breast cancers cells) and *in vivo* (in tumor samples from breast cancer patients). Moreover, survival analyses demonstrated that low expression levels of moesin were associated with a shorter overall survival specifically among patients diagnosed with a HER2+ breast cancer. Therefore, moesin is a predictive biomarker for diagnosing and/or prognosticating HER2+ breast cancer in a subject. Furthermore, the datas show that the loss of MSN is specific to HER2+ BC. The loss of MSN in a patient with BC is indicative of HER2+ cancer.

## Claims

1. A method for diagnosing and/or prognosticating HER2-dependent cancer in a subject, comprising:
a) measuring the amount of moesin in a sample from the subject;
b) comparing the amount of moesin measured in step a) to a reference value;
c) finding a deviation or no deviation of the amount of moesin measured in step a) from the reference value; and
d) attributing said finding of deviation or no deviation to a particular diagnosis and/or prognosis of HER2-dependent cancer in the subject;
wherein the HER2-dependent cancer is HER2+ breast cancer.

2. The method according to claim 1, wherein a lower amount of moesin in the sample from the subject compared to the reference value indicates that the subject has HER2-dependent cancer and/or indicates a short-term survival of said subject.

3. The method according to claim 1, wherein a higher amount of moesin in the sample from the subject compared to the reference value indicates that the subject has no HER2-dependent cancer or indicates a long-term survival of said subject.

4. The method according to any one of claim 1 to 3, wherein the sample is a tumor sample or a body fluid sample.

5. The method according to claim 4, wherein the body fluid sample is selected from the group consisting of of blood, serum plasma, saliva, urine, amniotic fluid, breast milk, bronchial lavage, cerebrospinal fluid, peritoneal fluid, seminal fluid, tears and pleural fluid, preferably blood.

6. The method according to claim 2, wherein the amount of the moesin in the sample from the subject is at least 0.5 fold decreased compared to the reference value, more preferably at least 0.7 fold decreased.

7. The method according to claim 3, wherein the amount of moesin in the sample from the subject is at least 1.5 fold increased compared to the reference value, more preferably at least 2 fold increased.

8. The method according to claim 1, 4 or 5, wherein the amount of moesin in the sample from the subject is equal to the reference value.

9. The method according to any one of claim 1 to 8, wherein the reference value represents (i) a diagnosis of no HER2-dependent cancer or (ii) a diagnosis of HER2-dependent cancer, (iii) a good prognosis of HER2-dependent cancer or (iv) a bad prognosis of HER2-dependent cancer.

10. A method for monitoring a change in the diagnosis and/or prognosis of HER2+ breast cancer in a subject, comprising:
a) applying the method of any of claims 1 to 9 to the subject at one or more successive time points, whereby the diagnosis and/or prognosis of HER2+ breast cancer in the subject is determined at said successive time points;
b) comparing the diagnosis and/or prognosis of HER2+ breast cancer in the subject at said successive time points as determined in step a); and
c) finding the presence or absence of a change between the diagnosis of HER2+ breast cancer in the subject at said successive time points as determined in step a).

11. The method according to claim 10, wherein said change in the diagnosis and/or prognosis of HER2-dependent cancer in the subject is monitored in the course of a medical treatment of said subject.

12. The method according to any one of claim 10 or 11, wherein the medical treatment is a prophylactic treatment or a therapeutic treatment, preferably an antitumoral treatment.

13. Use of a kit comprising:
- means for measuring the amount of moesin in the sample from the subject; and
- a reference value of the amount of moesin or means for establishing said reference value, said reference value representing a known diagnosis and/or prognosis of HER2+ breast cancer
for determining the diagnostic and/or the prognosis of HER2+ breast cancer in a subject suffering thereof.

## Patentansprüche

1. Verfahren zum Diagnostizieren und/oder Prognostizieren von einem HER2-abhängigen Krebs bei einem Individuum, umfassend:
a) Messen der Menge an Moesin in einer Probe von dem Individuum,
b) Vergleichen der Menge an Moesin, die in Schritt a) gemessen wurde, mit einem Referenzwert,
c) Feststellen einer Abweichung oder keiner Abweichung der Menge an Moesin, die in Schritt a) gemessen wurde, von dem Referenzwert, und
d) Zuschreiben dieses Feststellens einer Abweichung oder keiner Abweichung einer bestimmten Diagnose und/oder Prognose von einem HER2-abhängigen Krebs bei dem Individuum,
wobei der HER2-abhängige Krebs HER2+-Brustkrebs ist.

2. Verfahren nach Anspruch 1, wobei eine geringere Menge an Moesin in der Probe von dem Individuum verglichen mit dem Referenzwert anzeigt, dass das Individuum einen HER2-abhängigen Krebs aufweist, und/oder ein kurzfristiges Überleben des Individuums anzeigt.

3. Verfahren nach Anspruch 1, wobei eine höhere Menge an Moesin in der Probe von dem Individuum verglichen mit dem Referenzwert anzeigt, dass das Individuum keinen HER2-abhängigen Krebs aufweist, und/oder ein langfristiges Überleben des Individuums anzeigt.

4. Verfahren nach einem der Ansprüche 1 bis 3, wobei die Probe eine Tumorprobe oder eine Körperfluidprobe ist.

5. Verfahren nach Anspruch 4, wobei die Körperfluidprobe aus der Gruppe ausgewählt ist, bestehend aus Blut, Serumplasma, Speichel, Urin, Fruchtwasser, Muttermilch, Bronchiallavage, Zerebrospinalflüssigkeit, Peritonealflüssigkeit, Samenflüssigkeit, Tränen und Pleuraflüssigkeit, vorzugsweise Blut.

6. Verfahren nach Anspruch 2, wobei die Menge an Moesin in der Probe von dem Individuum mindestens 0,5-fach verglichen mit dem Referenzwert verringert ist, bevorzugter mindestens 0,7-fach verringert ist.

7. Verfahren nach Anspruch 3, wobei die Menge an Moesin in der Probe von dem Individuum mindestens 1,5-fach verglichen mit dem Referenzwert verringert ist, bevorzugter mindestens 2-fach verringert ist.

8. Verfahren nach Anspruch 1, 4 oder 5, wobei die Menge an Moesin in der Probe von dem Individuum gleich dem Referenzwert ist.

9. Verfahren nach einem der Ansprüche 1 bis 8, wobei der Referenzwert (i) eine Diagnose von einem nicht HER2-abhängigen Krebs oder (ii) eine Diagnose von einem HER2-abhängigen Krebs, (iii) eine gute Prognose von einem HER2-abhängigen Krebs oder (iv) eine schlechte Prognose von einem HER2-abhängigen Krebs darstellt.

10. Verfahren zum Überwachen einer Veränderung der Diagnose und/oder Prognose von HER2+-Brustkrebs bei einem Individuum, umfassend:
a) Anwenden des Verfahrens nach einem der Ansprüche 1 bis 9 auf das Individuum zu einem oder mehreren aufeinanderfolgenden Zeitpunkten, wobei die Diagnose und/oder Prognose von HER2+-Brustkrebs bei dem Individuum zu den aufeinanderfolgenden Zeitpunkten bestimmt wird,
b) Vergleichen der Diagnose und/oder Prognose von HER2+-Brustkrebs bei dem Individuum zu den aufeinanderfolgenden Zeitpunkten, wie sie in Schritt a) bestimmt wurde(n), und
c) Feststellen des Vorliegens oder Nichtvorliegens einer Veränderung zwischen der Diagnose von HER2+-Brustkrebs bei dem Individuum zu den aufeinanderfolgenden Zeitpunkten, wie sie in Schritt a) bestimmt wurde.

11. Verfahren nach Anspruch 10, wobei die Veränderung der Diagnose und/oder Prognose von einem HER2-abhängigen Krebs bei dem Individuum im Laufe einer medizinischen Behandlung des Individuums überwacht wird.

12. Verfahren nach einem der Ansprüche 10 oder 11, wobei die medizinische Behandlung eine prophylaktische Behandlung oder eine therapeutische Behandlung ist, vorzugsweise eine antitumorale Behandlung.

13. Verwendung eines Kits, umfassend:
- Mittel zum Messen der Menge an Moesin in der Probe von dem Individuum, und
- einen Referenzwert der Menge an Moesin oder Mittel zum Festlegen des Referenzwerts, wobei der Referenzwert eine bekannte Diagnose und/oder Prognose von HER2+-Brustkrebs darstellt,
zum Bestimmen der Diagnose und/oder der Prognose von HER2+-Brustkrebs bei einem Individuum, das darunter leidet.

## Revendications

1. Procédé pour diagnostiquer et/ou pronostiquer un cancer dépendant de HER2 chez un sujet, comprenant :
a) la mesure de la quantité de moésine dans un échantillon provenant du sujet ;
b) la comparaison de la quantité de moésine mesurée à l'étape a) avec une valeur de référence ;
c) la découverte d'un écart ou d'une absence d'écart de la quantité de moésine mesurée à l'étape a) par rapport à la valeur de référence ; et
d) l'attribution de ladite découverte d'un écart ou d'une absence d'écart à un diagnostic et/ou pronostic particulier de cancer dépendant de HER2 chez le sujet ;
dans lequel le cancer dépendant de HER2 est un cancer du sein HER2+.

2. Procédé selon la revendication 1, dans lequel une quantité de moésine dans l'échantillon provenant du sujet inférieure à la valeur de référence indique que le sujet a un cancer dépendant de HER2 et/ou indique une survie à court terme dudit sujet.

3. Procédé selon la revendication 1, dans lequel une quantité de moésine dans l'échantillon provenant du sujet supérieure à la valeur de référence indique que le sujet n'a pas de cancer dépendant de HER2 ou indique une survie à long terme dudit sujet.

4. Procédé selon l'une quelconque des revendications 1 à 3, dans lequel l'échantillon est un échantillon de tumeur ou un échantillon de fluide corporel.

5. Procédé selon la revendication 4, dans lequel l'échantillon de fluide corporel est choisi dans le groupe constitué par le sang, le plasma sérique, la salive, l'urine, le liquide amniotique, le lait maternel, un lavage bronchique, le liquide céphalo-rachidien, le fluide péritonéal, le fluide séminal, les larmes et le fluide pleural, de préférence le sang.

6. Procédé selon la revendication 2, dans lequel la quantité de moésine dans l'échantillon provenant du sujet est au moins 0,5 fois moindre que la valeur de référence, mieux encore au moins 0,7 fois moindre.

7. Procédé selon la revendication 3, dans lequel la quantité de moésine dans l'échantillon provenant du sujet est au moins 1,5 fois supérieure à la valeur de référence, mieux encore au moins 2 fois supérieure.

8. Procédé selon la revendication 1, 4 ou 5, dans lequel la quantité de moésine dans l'échantillon provenant du sujet est égale à la valeur de référence.

9. Procédé selon l'une quelconque des revendications 1 à 8, dans lequel la valeur de référence représente (i) un diagnostic d'absence de cancer dépendant de HER2 ou (ii) un diagnostic de cancer dépendant de HER2, (iii) un bon pronostic de cancer dépendant de HER2 ou (iv) un mauvais pronostic de cancer dépendant de HER2.

10. Procédé pour surveiller un changement de diagnostic et/ou pronostic de cancer du sein HER2+ chez un sujet, comprenant :
a) l'application du procédé de l'une quelconque des revendications 1 à 9 au sujet à un ou plusieurs points de temps successifs, moyennant quoi le diagnostic et/ou pronostic de cancer du sein HER2+ chez le sujet est déterminé auxdits points de temps successifs ;
b) la comparaison du diagnostic et/ou pronostic de cancer du sein HER2+ chez le sujet auxdits points de temps successifs comme déterminé à l'étape a) ; et
c) la découverte de la présence ou de l'absence d'un changement de diagnostic de cancer du sein HER2+ chez le sujet auxdits points de temps successifs comme déterminé dans l'étape a).

11. Procédé selon la revendication 10, dans lequel ledit changement de diagnostic et/ou pronostic de cancer dépendant de HER2 chez le sujet est surveillé au cours d'un traitement médical dudit sujet.

12. Procédé selon l'une quelconque des revendications 10 à 11, dans lequel le traitement médical est un traitement prophylactique ou un traitement thérapeutique, de préférence un traitement antitumoral.

13. Utilisation d'une trousse comprenant :
- des moyens pour mesurer la quantité de moésine dans l'échantillon provenant du sujet ; et
- une valeur de référence de la quantité de moésine ou des moyens pour établir ladite valeur de référence, ladite valeur de référence représentant un diagnostic et/ou pronostic connu de cancer du sein HER2+
pour déterminer le diagnostic et/ou le pronostic de cancer du sein HER2+ chez un sujet en souffrant.
